# EUROPÄISCHE PATENTANMELDUNG

(11) **EP 4 248 924 A1**
(43) Veröffentlichungstag der Anmeldung: **27.09.2023**
(21) Anmeldenummer: 22164545.0
(22) Anmeldetag: 25.03.2022
(51) Int. Cl.: A61F 9/008

(54) **VERFAHREN ZUR ERMITTLUNG VON BESTRAHLUNGSPARAMETERN SOWIE VORRICHTUNG ZUR BESTRAHLUNG**

(71) Anmelder: OD-OS GmbH, 14513 Teltow (DE)
(72) Erfinder: Schmidt, Eberhard, 14532 Kleinmachnow (DE); Rose, Arnd, 14532 Stahnsdorf (DE); Amthor, Kay-Uwe, 14513 Teltow (DE)
(74) Vertreter: Pfenning, Meinig & Partner mbB

(57) **Zusammenfassung**

Die Erfindung bezieht sich auf ein Verfahren zur Ermittlung von Bestrahlungsparametern für den Betrieb eines Lasers (2) zur Bestrahlung von einem oder mehreren Zielbereichen der Netzhaut (16) eines Auges (4) mit einem Behandlungsstrahl (11), bei dem die Netzhaut ganz oder teilweise mit einer Beleuchtungseinrichtung (5, 5') unter definierten Beleuchtungsparametern beleuchtet wird und während und/oder kurz nach der Beleuchtung wenigstens eine Aufnahme, insbesondere wenigstens ein Kamerabild, zumindest eines Zielbereiches der Netzhaut erfasst wird und bei dem unter Verwendung des/der Aufnahmen oder Kamerabilder zumindest Startwerte der Bestrahlungsparameter für die Bestrahlung eines oder mehrerer Zielbereiche der Netzhaut ermittelt werden. Zudem bezieht sich die Erfindung auf eine entsprechende Vorrichtung in Form eines erweiterten Ophthalmoskops und ermöglicht mit möglichst geringem Aufwand eine objektivierte Ermittlung von Startwerten für Bestrahlungsparameter bei einer Laserbehandlung einer Netzhaut.

## Beschreibung

Die Erfindung liegt auf dem Gebiet der medizinischen Messtechnik und bezieht sich konkret auf ein Verfahren zur Ermittlung von Bestrahlungsparametern zur Bestrahlung von Zielbereichen der Netzhaut eines Auges.

Es sind zur Behandlung verschiedener Augenkrankheiten Verfahren bekannt, bei denen durch eine Laserbehandlung der Netzhaut eine Gewebeveränderung durch sogenannte Laserkoagulation herbeigeführt werden kann. Bei allen Arten der Gewebebehandlung unter Verwendung eines Lasers ist es wichtig, im Vorhinein möglichst viel über die Wechselwirkung des verwendeten Laserstrahls mit dem Gewebe des individuellen Patienten in Erfahrung zu bringen.

Zu diesem Zweck ist es aus dem Stand der Technik bekannt, mit einem Laser sogenannte Titrationsschüsse abzusetzen, das heißt, bestimmte Gewebebereiche der Netzhaut einem Laserstrahl unter bestimmten Bedingungen auszusetzen, um experimentell die Wirkung einer bestimmten Intensität einer Laserbehandlung auf das Gewebe zu bestimmen. Der Nachteil dieser Methode liegt darin, dass einerseits gewisse Bereiche der Netzhaut ohne ein ausreichendes Vorwissen der Belastung durch eine Laserbehandlung ausgesetzt werden und dass andererseits zur Auswertung solcher Experimente eine gewisse Zeitdauer notwendig ist, da die Reaktion des Gewebes auf die experimentelle Laserbehandlung abgewartet werden muss. Dies dauert bei jedem Titrationsschuss zwar nur einige Sekunden, jedoch ist es üblich, eine Mehrzahl von Titrationsschüssen, insbesondere mit variabler Intensität, zu verwenden, um die Reaktion des Gewebes auf verschiedene Laserstärken zu ermitteln. Die Wirkung der Titration kann, falls sie unter der Sichtbarkeitsschwelle im Farbbild bleibt, auch durch andere Untersuchungsverfahren ermittelt werden.

Beispielsweise ist aus der WO 2011/038935 A1 ein Verfahren bekannt, bei dem im Zusammenhang mit Titrationsschüssen auf Bereiche einer Netzhaut mittels einer Kamera Bilder der Netzhaut erfasst werden, aus denen eine Verfärbung der durch den Laser getroffenen Gewebebereiche erkannt werden kann. Zudem wird ein Differenzbild zwischen vor der Titration und nach der Titration aufgenommenen Kamerabildern erzeugt, so dass aus Differenzwerten die Wirkung der Titration abgeleitet werden kann.

Der vorliegenden Erfindung liegt vor dem Hintergrund des Standes der Technik die Aufgabe zugrunde, ein Verfahren und eine Vorrichtung zur Ermittlung von Bestrahlungsparametern für den Betrieb eines Lasers zur Bestrahlung von einem oder mehreren Zielbereichen der Netzhaut eines Auges zu schaffen, mit denen die Bestrahlungsparameter mit möglichst geringem Zeitverlust und möglichst geringem Aufwand möglichst zuverlässig ermittelt werden können.

Die Aufgabe wird gemäß der Erfindung mit einem Verfahren mit den Merkmalen des Patentanspruchs 1 gelöst.

Demgemäß bezieht sich die Erfindung auf ein Verfahren zur Ermittlung von Bestrahlungsparametern für den Betrieb eines Lasers zur Bestrahlung von einem oder mehreren Zielbereichen der Netzhaut eines Auges mit einem Behandlungsstrahl, bei dem die Netzhaut ganz oder teilweise mit einer Beleuchtungseinrichtung unter definierten Beleuchtungsparametern beleuchtet wird und während und/oder kurz nach der Beleuchtung wenigstens eine Aufnahme, insbesondere wenigstens ein Kamerabild, zumindest eines Zielbereiches der Netzhaut erfasst wird und bei dem unter Verwendung des/der Aufnahmen oder Kamerabilder zumindest Startwerte der Bestrahlungsparameter für die Bestrahlung eines oder mehrerer Zielbereiche der Netzhaut ermittelt werden.

Mit dem Verfahren soll es ermöglicht werden, die vor der Behandlung stattfindende Ermittlung von Bestrahlungsparametern für den Betrieb eines Lasers möglichst schnell, möglichst weitgehend automatisiert und reproduzierbar durchzuführen. Gemäß dem Stand der Technik ist es oftmals üblich, dass experimentell Laserschüsse (Titrationsschüsse) auf Bereiche der Netzhaut aufgebracht werden, um die Reaktion des Gewebes bei dem individuellen Patienten zu ermitteln. Oft beurteilt der Operateur danach das Maß der Gewebeverfärbung infolge der Laserwirkung gemäß seinem persönlichen Eindruck und stellt darauf Parameter des Lasers für die Bestrahlung bei der eigentlichen Behandlung ein.

Mit dem erfindungsgemäßen Verfahren wird es möglich, auf einen Probebetrieb des Lasers vor der Behandlung vollständig zu verzichten oder diesen signifikant zu reduzieren. Durch die Verwendung von zeitnah gewonnenen Aufnahmen oder Kamerabildern der Netzhaut wird eine Beurteilung des individuell vorliegenden Netzhautgewebes objektiviert, transparent, nachvollziehbar, reproduzierbar und überprüfbar.

Es kann mit dem erfindungsgemäßen Verfahren ausreichen, auf dem beschriebenen Weg die Bestrahlungsparameter für den anschließenden Betrieb des Lasers zur Bestrahlung abschließend zu ermitteln, so dass die Netzhaut der Laserstrahlung ausschließlich zur Behandlung und zur experimentellen Feststellung der notwendigen Laserstärke im Rahmen eines Titrationsvorgangs ausgesetzt werden muss.

Als Beleuchtungseinrichtung kann eine Quelle für sichtbares Licht, beispielsweise eine Leuchtdiode oder ein Laser, beispielsweise auch der Behandlungslaser selbst, oder eine andere, breitbandige Lichtquelle zum Einsatz kommen. Auch eine Beleuchtung über die sichtbaren Bereiche des optischen Spektrums hinaus kann dabei in Betracht kommen. Unter den definierten Beleuchtungsparametern können in diesem Zusammenhang beispielsweise eine Lichtstärke und die Wellenlänge oder eine Wellenlängenverteilung der abgegebenen Strahlung verstanden werden. Diese können beispielsweise auf bestimmte Werte oder Charakteristiken eingestellt werden. Die Beleuchtungsparameter, wie beispielsweise die Beleuchtungsstärke, Verteilung der Beleuchtungsintensität sowie das Spektrum/die Wellenlängenverteilung können alternativ oder zusätzlich auch gemessen werden und die Messwerte können dann bei der Auswertung der Aufnahmen verwendet werden. Zudem ist anzumerken, dass auch Strahlung im nicht-sichtbaren Bereich, wie beispielsweise im infraroten Bereich, verwendet werden kann, wobei eine dabei erfasste Aufnahme oder ein Kamerabild in diesem Fall ebenfalls ein Infrarotbild ist, das mit entsprechenden Detektoren aufgenommen wird. Es können für die Aufnahmen beispielweise auch Hyperspektralsensoren eingesetzt werden. Für die Beleuchtung können auch bestimmte Bereiche der Netzhaut, beispielsweise Zielbereiche, automatisch oder manuell auswählbar sein. Bei der automatischen Auswahl können bestimmte geometrische Beleuchtungsmuster vorgegeben und auswählbar sein.

Als bildgebende Verfahren zur Erfassung einer Aufnahme oder eines Kamerabilds der Netzhaut können beispielsweise auch die Verfahren der Fundusfotografie, der Fundusreflektometrie, der Autofluoreszenzbildgebung, der fotoakustischen Bildgebung und der funktionellen optischen Kohärenztomographie mit entsprechenden Beleuchtungseinrichtungen und Beleuchtungsverfahren eingesetzt werden. Mit diesen Verfahren lässt sich beispielsweise die Pigmentierungsstärke der Netzhaut ermitteln, die für die Absorptionsstärke des später verwendeten Behandlungsstrahls maßgeblich ist. Die Pigmentierungsstärke kann jedoch auch allgemein aus der Helligkeit der Netzhaut, die mit der Aufnahme beziehungsweise dem Kamerabild erfasst wird, ermittelt werden.

Oft sollen nur bestimmte einzelne oder mehrere Zielbereiche der Netzhaut mit einem Laserstrahl behandelt werden. In diesem Fall kann es ausreichen, die Zielbereiche zu beleuchten und eine Aufnahme oder ein Kamerabild der Zielbereiche zu erfassen. Es kann jedoch auch sinnvoll sein, Aufnahmen/ Kamerabilder der gesamten Netzhaut zu erfassen, da die notwendige Bestrahlungsintensität des Lasers bei der Behandlung unter Berücksichtigung der Pigmentierungsstärke auch in anderen, nicht zur Behandlung vorgesehenen Bereichen der Netzhaut abgeschätzt werden kann. Auch andere, zusätzliche nichtlokale Eigenschaften der zu behandelnden Person können bei der Ermittlung der notwendigen Bestrahlungsintensität miteinbezogen werden. Zu diesem Thema werden weiter unten noch weitere Ausführungen gemacht.

Eine besondere Implementierung des oben beschriebenen Verfahrens kann auch vorsehen, dass zur Ermittlung der Startwerte der Bestrahlungsparameter mittels der Aufnahme(n) oder des/der Kamerabilder die Intensität der Pigmentierung für einen oder mehrere Zielbereiche der Netzhaut und/oder die Intensitätsverteilung der Pigmentierung auf der Netzhaut ermittelt werdenEs kann zudem vorgesehen sein, dass zur Ermittlung der Startwerte der Bestrahlungsparameter für einen oder mehrere Zielbereiche der Netzhaut zusätzlich Korrekturwerte bezüglich der zu erwartenden Absorption des Behandlungsstrahls im Glaskörper des Auges auf dem Weg zu den zu bestrahlenden Zielbereichen ermittelt werden.

Beispielsweise kann es vorteilhaft sein, die Melaninpigmentierung der Netzhaut zu erfassen und zu diesem Zweck die Netzhaut mit Licht im sichtbaren Bereich mit Wellenlängen oder sogar ausschließlich mit Wellenlängen zu bestrahlen, die von der Melaninpigmentierung besonders gut absorbiert werden.

Dabei können für eine Messung Wellenlängen der Beleuchtung und/ oder der Detektion im Bereich zwischen 450 nm und 1064 nm vorteilhaft sein. Insbesondere können Messungen bei Sonst 517nm, 532nm oder 577nm vorteilhaft sein. Die Streuung und Absorption der Strahlung durch Melanin ist in diesem Bereich stark. Zudem können auch die Wellenlängen vorteilhaft sein, bei denen die Unterschiede zwischen den Absorptionsstärken von Melanin und Blut signifikant sind. Es kann sich ein Vergleich von Aufnahmen bei Strahlung mit einer Wellenlänge von 480 nm (lokales Minimum der Absorption in Blut) mit Aufnahmen bei 550 nm (lokales Maximum der Absorption in Blut) anbieten. Als Referenzwerte bei der Reflexionsmessung können beispielsweise die Papille als Nervenknoten verwendet werden, die im gesunden Zustand eine starke Reflexion zeigt oder Bereiche mit Blutgefäßen, in denen die Anwesenheit von sauerstoffreichem oder sauerstoffarmem Blut selektiv nachweisbar ist und bei Verwendung von geeigneten Wellenlängen, die diese Blutvarianten nachweisen können, brauchbare Vergleichswerte erzeugt werden können.

Bei der nachfolgenden Laserbehandlung der Netzhaut wird der Behandlungsstrahl wegen der nicht hundertprozentigen Transparenz des Glaskörpers des Auges teilweise absorbiert oder gestreut. Dieser Effekt soll bei der Bestimmung der Bestrahlungsparameter für den Laser berücksichtigt werden, derart, dass die Laserintensität gegebenenfalls zur Kompensation etwas höher gewählt wird, als es für die Behandlung der Netzhaut unmittelbar notwendig ist. Die Absorption des Behandlungsstrahls hängt dabei von der individuellen Ausprägung des Glaskörpers bei dem zu behandelnden Patienten ab. Im Rahmen des erfindungsgemäßen Verfahrens kann die Absorption abgeschätzt werden, da die Beleuchtungsstärke, mit der die Netzhaut beleuchtet wird, bekannt ist und das rückgestreute Signal, beispielsweise das reflektierte Licht, bezüglich seiner Intensität sowohl von der Pigmentierungsstärke der Netzhaut als auch von der Absorption des Lichts im Glaskörper abhängt. Um die beiden die Rückstreuung beeinflussenden Effekte voneinander trennen zu können, können mehrere Messungen bei geänderten optischen Bedingungen durchgeführt werden. Genaueres hierzu wird weiter unten noch ausgeführt.

In vielen Fällen kann es sinnvoll sein, dass zur Erzeugung eines Kamerabildes die Netzhaut mit einer Strahlung mit bekannter Wellenlängenverteilung beleuchtet wird, die insbesondere eine Infrarotstrahlung und/oder Licht in einem durch Melanin absorbierten Wellenlängenbereich umfasst, wobei zusätzlich die Lichtstärke der Beleuchtungseinrichtung und/oder die auf eine Flächeneinheit der Netzhaut auftreffende gesamte Lichtleistung und/oder die auf eine Flächeneinheit der Netzhaut auftreffende Lichtleistung in einem definierten Wellenlängenbereich bestimmt wird und wobei die Helligkeitswerte und/oder Farben des Kamerabildes zur Ermittlung der Startwerte der Parameter für die Bestrahlung der Netzhaut verwendet werden.

Aus Erfahrungswerten für die notwendige Intensität des Behandlungsstrahls im Zusammenhang mit vorher gemessenen Helligkeitswerten der Netzhaut können für den Einzelfall Startwerte der Bestrahlungsparameter ermittelt werden, wobei beispielsweise die Absorption des Behandlungsstrahls durch den Glaskörper durch eine angenommene konstante Größe repräsentiert werden kann, die beispielsweise unter anderem vom Alter des Patienten abhängen kann.

Zudem kann vorteilhaft vorgesehen sein, dass für einen oder mehrere Zielbereiche die Dicke der Netzhaut ermittelt und bei der Ermittlung der Startwerte der Bestrahlungsparameter berücksichtigt wird, wobei die Ermittlung der Dicke der Netzhaut insbesondere durch die Erstellung einer OCT-Dickenkarte der Netzhaut erfolgt, und/oder dass zur Ermittlung der Startwerte der Bestrahlungsparameter die Art und Intensität der Pigmentierung von von der Netzhaut verschiedenen Körperregionen der zu behandelnden Person, insbesondere die Art und Intensität der Pigmentierung der Iris des Auges oder der Haut oder der Haare, verwendet werden.

Mittels einer OCT-Messung (optische Kohärenztomographie) kann die Dicke der Netzhaut ortsabhängig ermittelt werden, um beispielsweise Ödeme in der Netzhaut zu detektieren, die oft selbst einen Teil der Laserstrahlung absorbieren, so dass bei der eigentlichen Behandlung bestimmte Anteile des Behandlungsstrahls die Pigmentschicht nicht erreichen. Somit kann durch die OCT-Dickenkarte für die Bestrahlungsparameter eine ortsabhängige Korrekturgröße über die Gesamtfläche oder Teilflächen der Netzhaut ermittelt werden.

Es kann in einer anderen Implementierung des beschriebenen Verfahrens auch vorgesehen sein, dass zur Ermittlung der Startwerte der Bestrahlungsparameter außer der/den erfassten Aufnahmen oder Kamerabildern zusätzlich eine oder mehrere vorgegebene oder vorgebbare Referenzaufnahmen oder Referenzbilder verwendet werden.

Die Referenzaufnahmen oder Referenzbilder können beispielsweise Sollzustände der Netzhaut darstellen, die mit den erfassten Aufnahmen/Kamerabildern verglichen werden können, um eine Differenzwertanalyse durchzuführen und aus den Differenzen zwischen dem erfassten Bild und dem Referenzbild eine notwendige Bestrahlungsintensität beziehungsweise die notwendigen Bestrahlungsparameter oder deren Startwerte zu ermitteln. Die Bestrahlungsparameter, die die Behandlungsintensität oder Bestrahlungsintensität bestimmen, können typischerweise die Stärke des Laserstrahls, das heißt seine Energie, und seine Querschnittsfläche im Auftreffbereich/Zielbereich beziehungsweise die Größe des Laserstrahlflecks sowie die Zeitdauer und Anzahl abgegebener Laserpulse und Pausen zwischen den Laserpulsen umfassen. Letztlich können Referenzaufnahmen auch beispielsweise aus Datenbanken entnommen werden oder aus früher aufgenommenen Aufnahmen/Kamerabildern desselben Patienten gewonnen werden.

In einer weiteren Implementierung des oben beschriebenen Verfahrens kann außerdem vorgesehen sein, dass zur Ermittlung der Bestrahlungsparameter oder zumindest von Startwerten der Bestrahlungsparameter für einen oder mehrere zu bestrahlende Zielbereiche der Netzhaut unabhängig voneinander einerseits die Intensität der Pigmentierung des/der Zielbereich(e) und andererseits die zu erwartende Absorption des Behandlungsstrahls in der Augenlinse auf dem Weg zu den Zielbereichen ermittelt werden, indem wenigstens zwei Messungen unter verschiedenen Beleuchtungsbedingungen durchgeführt werden, wobei bei den zwei oder mehr Messungen insbesondere wenigstens einer der folgenden Parameter variiert wird: Größe des fokussierten Lichtflecks auf der Netzhaut, wobei jeweils die Abweichung von einer zu erwartenden Lichtintensitätsverteilung auf der Fläche des Fokusflecks gemessen wird, Einstrahlrichtung eines Beleuchtungsstrahls der Beleuchtungseinrichtung durch die Pupille und den Glaskörper zur Beleuchtung des jeweiligen Zielbereiches, Einstrahlrichtung zum Zielbereich und/oder Ausfallrichtung eines erfassten reflektierten Beleuchtungsstrahls der Beleuchtungseinrichtung, Wellenlängenbereich oder Wellenlängenverteilung eines Beleuchtungsstrahls der Beleuchtungseinrichtung.

Dieses Verfahren lässt die Ermittlung der Verteilung der Pigmentierungsstärke auf der Netzhaut einerseits und die Ermittlung der Absorption eines Laserstrahls durch den Glaskörper in Abhängigkeit vom Laserzielpunkt auf der Netzhaut und vom Lichtweg andererseits unabhängig voneinander zu. Für eine solche Messung kann beispielsweise bei verschiedenen aufeinanderfolgenden Einzelmessungen der Lichtweg bei der Beleuchtung eines Zielbereichs der Netzhaut geändert, jedoch jeweils derselbe Zielbereich beleuchtet und aufgenommen werden. Andererseits kann auch in mehreren Messungen ein Zielbereich der Netzhaut durch die Beleuchtungseinrichtung bei gleichbleibendem Lichtweg in unterschiedlicher Weise beleuchtet werden, so dass der Einfluss der Reflektivität der Netzhaut durch Beleuchtung mit unterschiedlichen Spektren beziehungsweise durch Licht in verschiedenen Wellenlängenbereichen variiert werden kann.

Es kann alternativ oder zusätzlich dazu auch vorgesehen sein, dass zur Ermittlung der Bestrahlungsparameter für einen oder mehre-re zu bestrahlende Zielbereiche der Netzhaut einerseits die Intensität der Pigmentierung des/der Zielbereich(e) und/oder die zu erwartende Absorption des Behandlungsstrahls in der Augenlinse auf dem Weg zu den Zielbereichen ermittelt werden, indem wenigstens zwei Messungen durchgeführt werden, wobei eine der Messungen auf die - und/oder Reflektionscharakteristik im Bereich der Papille und/oder wenigstens eines Blutgefäßes oder eines Teils eines Blutgefäßes in der Netzhaut gerichtet ist, während eine zweite Messung auf die Absorptions- und/oder Reflektionscharakteristik in einem der übrigen zu bestrahlenden Bereiche der Netzhaut gerichtet ist.

Der optische Nervenkopf (Papille) ist üblicherweise nicht pigmentiert und reflektiert das Licht wesentlich stärker als die pigmentierten Teile der Netzhaut. Die Papille kann somit als "Reflektionsnormal" oder Referenz für eine Vergleichsmessung der Intensität der Pigmentierung dienen;
Die Adern der Netzhaut können ebenfalls detektiert und für eine Vergleichsmessung in den durch sie definierten Bereichen der Netzhaut herangezogen werden. Das geführte Blut hat eine bestimmte andere Absorptionscharakteristik als das Melanin. Die Absorption hängt auch davon ab, ob das Blut mit Sauerstoff gesättigt ist oder nicht, das heißt, welche Art von Gefäßen herangezogen wird. Um diese Eigenschaften der Absorption und Reflektivität bei den Blutgefäßen differenziert messen und auswerten zu können, kann es sinnvoll sein, eine Reflektivitäts- oder Absorptionsmessung bei Wellenlängen des Beleuchtungslichts durchzuführen, die gerade besonders stark durch das Sauerstoff-gesättigte oder das sauerstoffarme Blut absorbiert werden. Alternativ zur entsprechenden Auswahl des Beleuchtungslichts können auch auf der Detektionsseite die entsprechenden aussagekräftigen Wellenlängen selektiert werden.

Durch eine Mehrzahl von Messungen kann in einer Auswertung danach der Einfluss der Pigmentierung auf die erfasste Aufnahme beziehungsweise das erfasste Kamerabild einerseits und der Einfluss der Absorption des Glaskörpers andererseits unabhängig voneinander ermittelt und bei der Bestimmung der Bestrahlungsparameter berücksichtigt werden. Ergänzend soll noch festgehalten werden, dass mit der Einstrahlrichtung und der Ausfallrichtung des Beleuchtungsstrahls jeweils auch der Lichtweg, den die Laserstrahlung durch den Glaskörper hindurch zurücklegt, gemeint ist.

Die Art der Verknüpfung von Beleuchtungsparametern und den detektierten Größen/Signalen der jeweils erfassten Aufnahme oder des jeweils erfassten Kamerabilds einerseits und den Bestrahlungsparametern für den Betrieb des Lasers andererseits kann in vielfältiger Gestalt vorliegen. Im einfachsten Fall kann dies eine Zuordnungstabelle sein, die als mehrdimensionale Matrix vorliegen kann und die beispielsweise in einem Computer oder einer nicht lokalen oder verteilten Datenverarbeitungsanlage gespeichert sein kann. Die Verknüpfung kann auch in Form eines Algorithmus oder eines neuronalen Netzes vorliegen, der beispielsweise durch selbstlernende oder geführt lernende Verfahren trainiert sein kann.

Vor diesem Hintergrund kann sich die vorliegende Erfindung auch auf ein Verfahren zum Training eines Algorithmus oder eines neuronalen Netzes zur Ermittlung der Startwerte von Bestrahlungsparametern gemäß dem oben beschriebenen Verfahren beziehen, wobei für eine Mehrzahl von Einzelbehandlungen als Trainingsdaten einerseits aus Bildern ermittelte Datensätze von zu bestrahlenden Zielbereichen und die zugehörigen Beleuchtungsparameter sowie insbesondere jeweils zusätzlich erfasste Sekundärinformationen als Eingangsinformationen und andererseits die bei der jeweils nachfolgenden Laserbehandlung eingestellten Bestrahlungsparameter als Ergebnisgrößen miteinander verknüpft werden.

Die Trainingsdaten können beispielsweise anfangs durch einen Parallelbetrieb aus einem herkömmlichen Verfahren und dem hier geschilderten Verfahren gewonnen werden, wobei jeweils, wie oben beschrieben, unter definierten Beleuchtungsbedingungen eine Aufnahme oder ein Kamerabild erfasst wird und darauf in konventioneller Weise mit Titrationsverfahren die notwendige Behandlungsintensität, das heißt die Intensität des Behandlungslaserstrahls, z. B. repräsentiert durch Laserleistung, Pulsdauer, Pulszahl und Wiederholrate festgestellt wird. Diese kann dann für die Zielbereiche der Netzhaut, die zu behandeln sind, mit den vorher aufgenommenen Aufnahmen/Kamerabildern verknüpft werden, so dass das lernende System mit diesen Daten trainiert wird.

Die Einzelbehandlungen, anhand deren der Algorithmus trainiert wird, können auch bereits nach dem oben beschriebenen erfindungsgemäßen Verfahren durchgeführt werden, wobei zunächst die Startwerte für Bestrahlungsparameter ermittelt und dann die Behandlung begonnen wird und wobei darauf durch Kontrolle der Verfärbung der Zielbereiche der Erfolg der Behandlung beurteilt und gegebenenfalls die Bestrahlungsparameter korrigiert werden. Durch Eingabe der Korrekturwerte beziehungsweise Verknüpfung der Korrekturwerte mit den Parametern der Beleuchtungsstärke und den anfangs ermittelten Startwerten für die Bestrahlungsparameter kann der Algorithmus zur Verbesserung weiter trainiert werden.

Als zusätzlich erfasste Sekundärinformationen können Größen eingegeben werden, die sich als einflussreich für die Ermittlung der Bestrahlungsparameter herausstellen. Solche Sekundärinformationen können beispielsweise das Alter des Patienten und bestimmte Parameter seines Gesundheitszustands sowie speziell besondere Parameter der zu behandelnden Netzhaut des Patienten sein. Beispielsweise kann eine gemessene integrale Pigmentierungsstärke der Haut oder der Iris des Auges oder eine gemessene Pigmentierungsstärke des Haupthaars, der Haare der Augenbrauen oder der Haut des Patienten sowie die Farbe der Hautpigmentierung, die Farbe der Iris und die Farbe der Haare berücksichtigt werden.

Es kann beispielsweise auch vorgesehen sein, dass bei der Ermittlung der Startwerte der Bestrahlungsparameter für alle Zielbereiche eine Kalibrierung mit einem oder mehreren der folgenden Kalibrierparameter der jeweils zu behandelnden Person durchgeführt wird: gemessene integrale Pigmentierungsstärke der Haut, gemessene integrale Pigmentierungsstärke der Iris des Auges, gemessene Pigmentierungsstärke der Haare (Haupthaare, Augenbrauen), Farbe der Hautpigmentierung, Farbe der Iris, Farbe der Haare. Eine solche Kalibrierung kann beispielsweise auch vorsehen, dass die aufgezählten Größen nicht in den Algorithmus zur Ermittlung der Bestrahlungsparameter eingehen, sondern dass nach Ermittlung einer Bestrahlungsintensität eine Kalibrierung der Ergebnisse mit den genannten Größen durchgeführt wird, indem beispielsweise die Bestrahlungsintensität, gegeben durch die Laserenergie und die Länge des Laserpulses, mit einem bestimmten Kalibrierfaktor multipliziert wird.

Die Erfindung kann sich auch auf ein Verfahren zur Ermittlung von Bestrahlungsparametern für den Betrieb eines Lasers zur Bestrahlung von einem oder mehreren Zielbereichen der Netzhaut eines Auges mit einem Behandlungsstrahl beziehen, bei dem zunächst Startwerte der Bestrahlungsparameter nach dem oben beschriebenen Verfahren ermittelt werden, bei dem darauf mindestens ein Zielbereich mit einem Titrationsstrahl oder einem Behandlungsstrahl mit den Startparametern bestrahlt wird, bei dem dann wenigstens eine Fortsetzungsaufnahme oder ein Fortsetzungskamerabild des oder der mit dem Behandlungsstrahl bestrahlten Zielbereiche der Netzhaut erfasst wird und bei dem unter Verwendung des/der Fortsetzungsaufnahmen und/oder des/der Fortsetzungskamerabilder und der Startwerte der Bestrahlungsparameter zumindest Fortsetzungswerte der Bestrahlungsparameter für die Bestrahlung eines oder mehrerer Zielbereiche der Netzhaut ermittelt werden.

Auf diese Weise wird es ermöglicht, die anfangs ermittelten Startwerte der Bestrahlungsparameter möglichst bald nach ihrer Anwendung auf den erzielten Erfolg zu überprüfen und gegebenenfalls anzupassen oder aus diesen Fortsetzungsbestrahlungsparameter zu ermitteln. Die entsprechenden Korrekturen können beispielsweise auch dazu dienen, den Algorithmus mit weiteren Trainingsdaten zu versorgen und damit zu verbessern.

Letztlich bezieht sich die Erfindung außer auf das oben beschriebene Verfahren zudem noch auf eine Vorrichtung zur Bestrahlung von einem oder mehreren Zielbereichen der Netzhaut eines Auges mit einem Behandlungsstrahl, mit einem Laser zur Erzeugung des Behandlungsstrahls, mit einer Beleuchtungseinrichtung zur Beleuchtung von wenigstens einem oder mehreren Zielbereichen der Netzhaut mit definierten Beleuchtungsparametern sowie mit einer Aufnahmeeinrichtung, insbesondere einer Kamera, zur Erfassung wenigstens einer Aufnahme oder eines Bildes zumindest eines Zielbereiches der Netzhaut und mit einer Verarbeitungseinrichtung, die dazu eingerichtet ist, unter Verwendung der Beleuchtungsparameter und des/der Aufnahmen oder Kamerabilder zumindest Startwerte der Bestrahlungsparameter für die Bestrahlung eines oder mehrerer Zielbereiche der Netzhaut mit einem Behandlungsstrahl zu ermitteln.

Diese Vorrichtung kann zur Durchführung der verschiedenen Implementierungen des oben beschriebenen Verfahrens dienen. Die Verarbeitungseinrichtung kann dazu als Datenverarbeitungseinrichtung ausgebildet sein, die einen Algorithmus enthält, welcher den Beleuchtungsparametern und den mit der Aufnahme oder dem Kamerabild erfassten Größen die Startwerte für Bestrahlungsparameter zuordnet. Dieser Algorithmus kann entweder festgelegt sein oder im Rahmen eines lernenden Systems mit künstlicher Intelligenz mit Trainingsdaten trainiert sein, wie dies weiter oben bereits beschrieben wurde. Die Verarbeitungseinrichtung kann beispielsweise auch ein neuronales Netz enthalten, das in der Lage ist, in der beschriebenen Weise zu lernen, indem es durch den Trainingsvorgang seine Verknüpfungen bildet.

Die genannte Vorrichtung kann beispielsweise vollständig unabhängig von der Behandlungseinrichtung mit dem Behandlungslaser sein oder auch mit der Behandlungseinrichtung verbunden oder in diese integriert sein. Beispielsweise kann die erfindungsgemäße Vorrichtung auch teilweise zentralisiert sein, wobei die Verarbeitungseinrichtung nichtlokal in einem Server oder einer entfernten Datenverarbeitungsanlage anderer Art angeordnet sein kann und die ermittelten Daten durch ein geeignetes Kommunikationsverfahren erhält. Die Verarbeitungseinrichtung kann auf diese Weise mit Messdaten von vielen verschiedenen Orten gespeist und trainiert werden, und sie kann lokale Behandlungseinrichtungen aus der Entfernung steuern. Auf diese Weise kann jeweils ein optimales Training der Verarbeitungseinrichtung mit tatsächlichen Daten von einem oder mehreren Patienten gewährleistet werden.

Im Folgenden wird die Erfindung anhand von Figuren in Ausführungsbeispielen gezeigt und nachfolgend erläutert. Dabei zeigt
- Fig. 1: ein Ophthalmoskop mit einer Vorrichtung zur Bestrahlung der Netzhaut eines Auges,
- Fig. 2: ein Ablaufdiagramm zu einem Verfahren zur Ermittlung von Startwerten für Bestrahlungsparameter,
- Fig. 3: eine Abbildung von Zielbereichen einer Netzhaut,
- Fig. 4: eine vergrößerte Darstellung des Lichtwegs durch den Glaskörper eines Auges zur Netzhaut,
- Fig. 5: ein Beispiel für Beleuchtungsparameter, Messwerte, die eine Aufnahme der Zielbereiche einer Netzhaut repräsentieren, sowie zugeordnete Startwerte für Bestrahlungsparameter,
- Fig. 6: ein zweites Ablaufdiagramm eines Verfahrens, bei dem zunächst Startwerte für Bestrahlungsparameter bestimmt und diese im weiteren Verlauf überprüft werden,
- Fig. 7: ein drittes Ablaufdiagramm, das ein Verfahren zum Trainieren eines Algorithmus darstellt, der in einem selbstlernenden Verfahren vervollkommnet wird und eine Zuordnung der Startwerte von Bestrahlungsparametern zu den Beleuchtungsparametern und sonstigen Korrekturgrößen leistet, sowie
- Fig. 8: schematisch die Verknüpfung von Eingangsgrößen und Ausgangsgrößen für das Trainieren des Algorithmus.

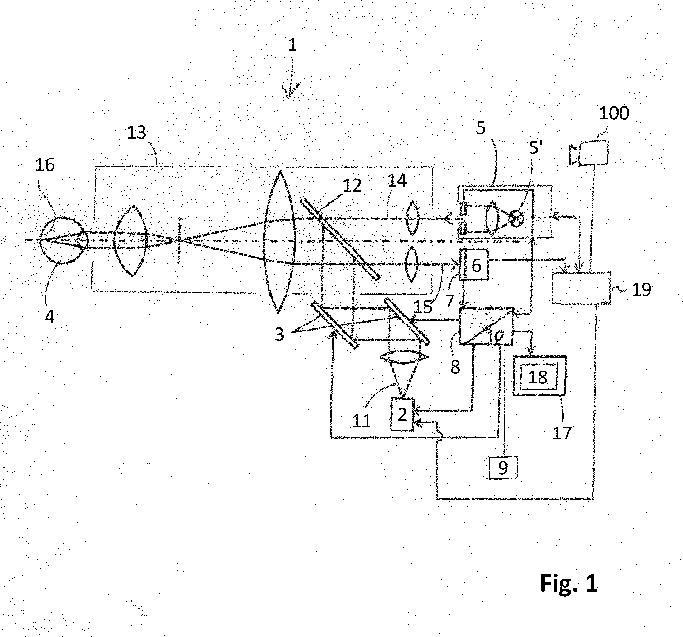

Figur 1 zeigt eine erfindungsgemäße Vorrichtung beziehungsweise eine Vorrichtung zur Durchführung des erfindungsgemäßen Verfahrens in Form eines Ophthalmoskops beziehungsweise eine Vorrichtung, die Funktionen eines Ophthalmoskops mit Funktionen der Laserbehandlung der Netzhaut verbindet.

Die Vorrichtung weist eine Beleuchtungseinrichtung 5 mit einer Strahlungsquelle 5' auf, die dazu eingerichtet ist, einen Beleuchtungsstrahl 14 auf das Auge 4 und die Netzhaut 16 eines Patienten zu richten. Damit kann die Netzhaut 16 zur Erfassung einer Aufnahme beziehungsweise eines Kamerabilds geeignet beleuchtet werden. Die Beleuchtung kann beispielsweise mit einer Leuchtdiode oder einer Infrarotdiode als Lichtquelle oder mit einer Lichtquelle anderer Art ausgestattet sein, die ein definiertes Wellenlängenspektrum liefert. Die Lichtquelle kann beispielsweise auch eine UV-Lichtquelle sein.

Das Ophthalmoskop verfügt zudem über eine Kamera 6, bei der ein Sensor mit 7 bezeichnet ist. Der Sensor kann beispielsweise ein CCD- oder CMOS-Sensor sein. Anstelle der Kamera 6 kann auch jede andere Art von Einrichtung vorgesehen sein, die beispielsweise als Scanvorrichtung dazu geeignet ist, Strahlung, die von der Netzhaut reflektiert oder gestreut wird, zu detektieren.

Das Ziel beim Betrieb der Beleuchtungseinrichtung 5 und der Kamera 6 oder einer gleichwertigen Einrichtung besteht darin, unter definierten Beleuchtungsbedingungen durch Erfassung einer Aufnahme von rückgestrahlter Strahlung möglichst genaue, ortsaufgelöste Messdaten von der Netzhaut 16 zu erhalten und damit die Eigenschaften von zu behandelnden Zielbereichen zu erfassen beziehungsweise ermitteln zu können.

Der Beleuchtungsstrahl 14 und die zurückgeworfene Strahlung 15 werden durch ein geeignetes optisches System 13 mit Spiegeln und Linsen in an sich bekannter Weise geeignet kollimiert beziehungsweise fokussiert. Das optische System 13 weist zudem einen Strahlteiler 12 auf, der es ermöglicht, einen Laserstrahl von dem Behandlungslaser 2 auf die Netzhaut 16 zu richten. Alternativ kann der Laserstrahl auch ohne Strahlteiler eingekoppelt werden, beispielsweise indem er leicht seitlich versetzt gegenüber dem Beleuchtungslicht geführt wird. Zur Steuerung des Lasers 2 kann eine Steuereinheit 8 vorgesehen sein, die einerseits die Beleuchtungseinrichtung 5 steuert, beispielsweise triggert, andererseits ein Kamerabild von der Kamera 6 erfasst und unmittelbar den Laser 2 steuert. Die Steuereinheit 8 kann dabei auch Umlenkspiegel 3 steuern, die den Strahlengang des Behandlungsstrahls 11 lenken und damit gezielt die Behandlung einzelner Zielbereiche auf der Netzhaut 16 ermöglichen.

Zur verbesserten Steuerung des Lasers 2 ist gemäß der vorliegenden Erfindung eine Verarbeitungseinrichtung 19 vorgesehen, die eine genaue Verarbeitung von Aufnahmen/Kamerabildern der Kamera 6 erlaubt und diese mit den bekannten und definierten Parametern der Beleuchtung der Netzhaut 16 verknüpft.

Das Ophthalmoskop weist beispielhaft noch einen Sensor 100, beispielsweise in Form einer Kamera, auf, der die Messung der Pigmentierungsfarbe und -stärke der Haut, der Haupthaare und/oder der Iris des Patienten erlaubt. Es kann auch eine Eingabevorrichtung vorgesehen sein, mit der ein solcher Parameter eingegeben werden kann. In jedem Fall werden diese Parameter an die Verarbeitungsvorrichtung 19 gegeben und dort bei der Ermittlung der Startwerte für Bestrahlungsparameter berücksichtigt.

In der Vergangenheit wurde die Intensität der Laserbehandlung, das heißt die Stärke und/oder Dauer der Laserpulse, mit denen der Laser 2 betrieben wurde, nach Beurteilung einer Aufnahme der Netzhaut von einem Operateur nach eigener Einschätzung durchgeführt. Dabei wurden zunächst Titrationspulse auf die Netzhaut gerichtet und deren Wirkung beurteilt, um die Laserstärke zu skalieren.

Durch die Beleuchtung der Netzhaut mit bekannten Beleuchtungsparametern und deren Verknüpfung mit der Aufnahme der Netzhaut ist es ermöglicht, in objektivierter Weise durch die Verarbeitungseinrichtung 19 diesen teilweise vorgegebenen, teilweise gemessenen Werten eine Intensität der Laserbehandlung für jeden Zielbereich auf der Netzhaut zuzuordnen, wobei die Intensität durch die Energie des Lasers, die Größe des Laserflecks auf der Netzhaut und die Anzahl, Wiederholrate und Dauer des oder der Pulse sowie die Länge der Pausen zwischen den Pulsen gegeben ist. Mit dem erfindungsgemäßen Verfahren lassen sich zumindest Startwerte für eine derartige Bestrahlung durch den Behandlungsstrahl 11 ermitteln, mit denen die Behandlung der Netzhaut durchgeführt werden kann.

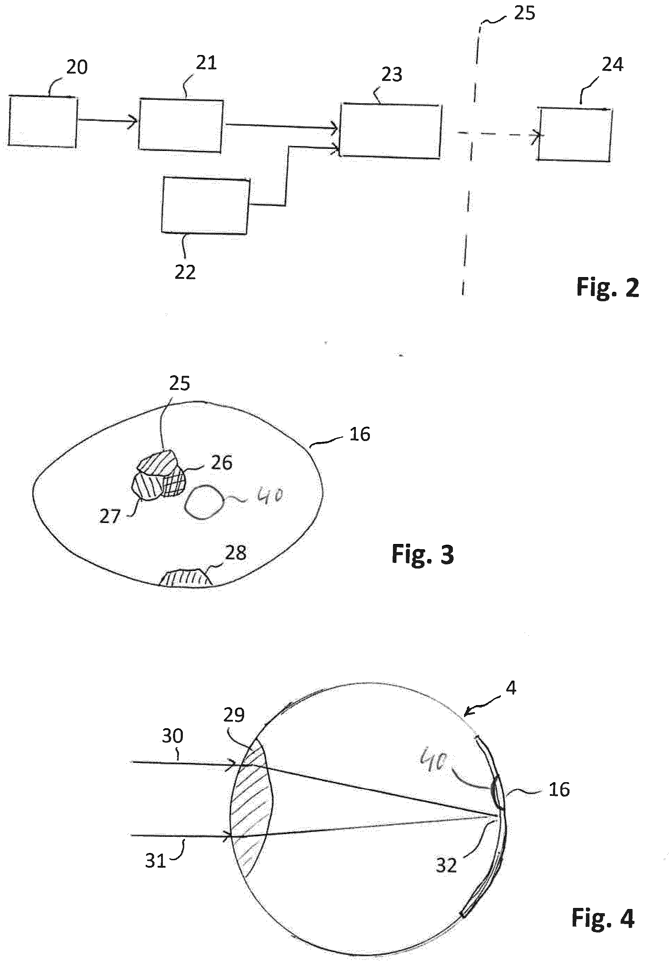

Figur 2 zeigt ein Ablaufdiagramm des erfindungsgemäßen Verfahrens, bei dem in einem ersten Schritt 20 Parameter der Beleuchtung der Beleuchtungseinrichtung 5 vorgegeben werden. Die Beleuchtungsparameter können dabei jedoch auch in der Beleuchtungseinrichtung bereits vorliegen und vorgegeben sein. Alternativ gibt es die Möglichkeit, dass individuelle Beleuchtungsparameter beispielsweise durch die Verarbeitungseinrichtung 19 vorgegeben und an die Beleuchtungseinrichtung 5 gesendet werden. Die Beleuchtungsparameter, wie beispielsweise die Beleuchtungsstärke, Verteilung der Beleuchtungsintensität sowie das Spektrum/die Wellenlängenverteilung können auch gemessen werden und die Messwerte können dann bei der Auswertung verwendet werden.

Darauf wird in einem zweiten Schritt 21 die Beleuchtungseinrichtung 5 beziehungsweise die Lichtquelle 5' betrieben und die Netzhaut entsprechend dauerhaft oder impulsartig beleuchtet. Gleichzeitig kann die rückgestrahlte Strahlung in einem weiteren Schritt 22 durch eine Kamera 6 aufgenommen werden. Der Schritt 22 kann gleichzeitig mit der Beleuchtung 21 oder auch zeitlich versetzt gegenüber der Beleuchtung durchgeführt werden, wenn beispielsweise eine Fluoreszenzstrahlung aufgenommen wird, die bekanntermaßen nach der Anregungsstrahlung nachleuchtet.

Die Beleuchtungsparameter und die Messwerte, die durch eine Kamera aufgenommen worden sind, werden dann in einem Schritt 23 gemeinsam in einer Verarbeitungseinrichtung 19 miteinander durch eine Zuordnungsfunktion, beispielsweise einen Zuordnungsalgorithmus, miteinander verknüpft, und es werden aus diesen Größen durch die Verknüpfung Startwerte für die Bestrahlungsparameter ermittelt, die durch den Betrieb des Lasers 2 in eine Bestrahlung umgesetzt werden sollen. Erst nachdem die Startwerte ermittelt sind, kann eine Behandlung 24 beginnen, die einen weiteren Schritt darstellt. Der Schritt 24 der eigentlichen Behandlung ist von der Ermittlung der Startwerte der Bestrahlungsparameter durch die gestrichelte Linie 25 in der Darstellung getrennt.

In Figur 3 ist schematisch ein Bild der Netzhaut 16 eines Auges mit verschiedenen Zielbereichen dargestellt. Es ist ein Zielbereich 28 am Rand der Netzhaut dargestellt, der für die eigentliche Sehleistung des Auges nicht so wichtig ist wie die Zielbereiche 25, 26, 27 im Zentrum der Netzhaut 16. Durch die verschiedenen Schraffuren der einzelnen Zielbereiche sollen verschiedene Pigmentierungsstärken oder andere variierende Parameter der einzelnen Zielbereiche repräsentiert werden. Dadurch soll deutlich werden, dass die verschiedenen Zielbereiche für die Behandlung durch den Laser 2 unterschiedliche Bestrahlungsparameter erfordern. Die Größe der Zielbereiche 25, 26, 27, 28 kann variieren und auch so klein sein, wie es eine ortsaufgelöste Behandlung durch einen Laser 2 ermöglicht. In den Figuren 3 und 4 ist jeweils auch die Papille dargestellt und mit 40 bezeichnet. Der Bereich der Papille bleibt unbehandelt und kann als Referenz für die Reflektivität im Vergleich zu den Melanin tragenden Bereichen der Netzhaut dienen.

Figur 4 zeigt einen Querschnitt durch den Glaskörper eines Auges 4 mit einer Augenlinse 29 und einer Netzhaut 16. Es ist auf der Netzhaut ein Punkt oder Zielbereich 32 angedeutet, der alternativ gezielt mit zwei verschiedenen Beleuchtungsstrahlen 30, 31 beleuchtet werden kann. Je nach der Eintrittsstelle des Beleuchtungsstrahls 30, 31 in die Augenlinse nimmt der Lichtstrahl unterschiedliche Wege durch die Linse und den Glaskörper zum Punkt 32. Werden die Messwerte des rückgestrahlten Lichts, die durch die Kamera 6 aufgenommen werden, bezüglich ihrer Intensität miteinander verglichen, so kann die Streuung beziehungsweise Dämpfung des Lichts auf den verschiedenen Lichtwegen durch den Glaskörper differenziert aufgenommen werden. Durch Variation der Beleuchtungsstrahlen kann der gesamte Glaskörper des Auges 4 nach und nach vermessen werden, so dass der Einfluss des Glaskörpers durch Dämpfung und Streuung auf die Beleuchtung der Netzhaut sowie den Behandlungsstrahl für alle Bereiche des Auges ermittelt werden kann.

Alternativ ist es auch möglich, zwei verschiedene Beleuchtungsstrahlen 30 auf demselben Lichtweg zu dem Punkt 32 auf der Netzhaut 16 gelangen zu lassen, jedoch beispielsweise die Wellenlänge des eingestrahlten Lichts zu variieren, so dass für beide Strahlen derselbe Lichtweg durch den Glaskörper vorliegt, jedoch beispielsweise einmal eine Wellenlänge gewählt wird, die der optimalen Absorptionswellenlänge von Melanin auf der Netzhaut entspricht, und in einem anderen Fall eine von dieser sich unterscheidende Wellenlänge. Damit kann die Pigmentierung mit Melanin auf der Netzhaut in erster Näherung unabhängig von Inhomogenitäten des Glaskörpers des Auges gemessen werden, wenn in einem ersten Schritt die wellenlängenabhängige Dämpfung des Glaskörpers vernachlässigt wird. Alternativ kann der Beleuchtungsstrahl auch ein breites Spektrum haben oder mehrere Wellenlängenbereiche gleichzeitig beinhalten. Der Sensor kann in diesem Fall durch wellenlängenselektive Auswertung über einen weiten Wellenlängenbereich- auch über den optisch sichtbaren Bereich hinaus, die Differenzierung leisten. Hierzu kann beispielsweise ein Hyperspectral - Sensor eingesetzt werden. Auch Bayer Filter können für die Aufnahme der Netzhaut eingesetzt werden.

Durch kombinierte Messungen, bei denen sowohl der Lichtweg als auch die Wellenlänge verändert wird, können dann auch die wellenlängenabhängigen Dämpfungswerte des Glaskörpers ortsabhängig ermittelt werden. Auf diese Weise ist es möglich, sobald die Parameter der Lichtquelle 5' bekannt sind, die Dämpfung sowohl des eingestrahlten Lichts oder der eingestrahlten Strahlung auf die Retina als auch den Einfluss des Lichtwegs bei der rückgestrahlten Strahlung zu berücksichtigen, so dass beispielsweise die Pigmentierungsstärke mit Melanin auf der Retina objektiviert ermittelt werden kann.

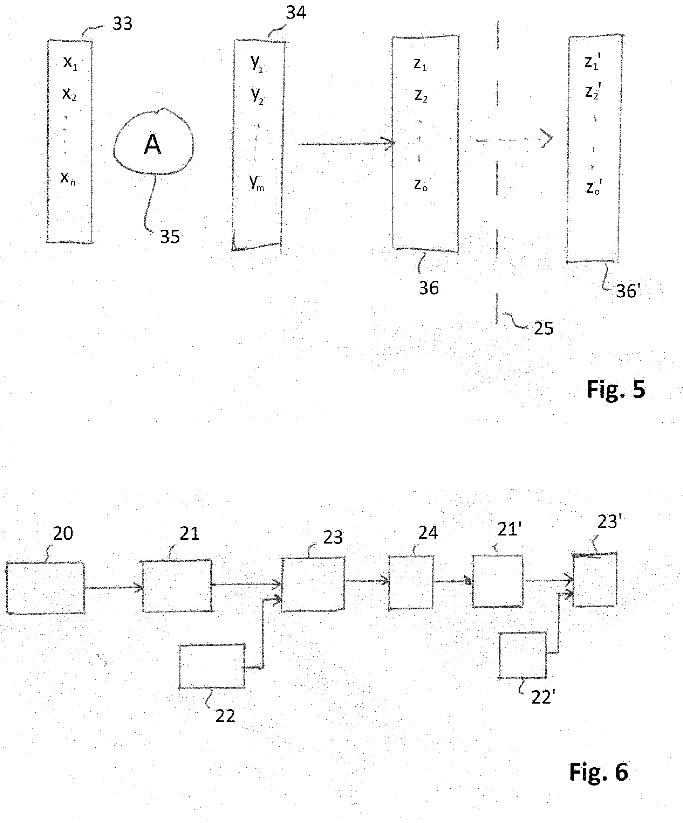

In Figur 5 ist schematisch die Struktur der Verknüpfung von Beleuchtungsparametern, Messwerten der Aufnahme / des Kamerabilds und den Startwerten für Bestrahlungsparameter dargestellt. Die Beleuchtungsparameter 33 sind mit x₁ bis xₙ bezeichnet, wobei die einzelnen Parameter beispielsweise Beleuchtungsstärken, das heißt die von der Beleuchtungsquelle abgestrahlte Strahlungsenergie oder Leistung, jeweils für bestimmte Zielbereiche bezeichnen, oder auch die Verteilung der Leistung auf einzelne Wellenlängenbereiche der abgegebenen Strahlung, das heißt die Gestalt eines Wellenlängenspektrums mit entsprechenden Intensitäten. Mit 35 ist die Abbildungsfunktion oder Verknüpfungsfunktion bezeichnet, die beispielsweise als selbstlernender Algorithmus gestaltet sein kann.

Mit 34 werden die Parameter y₁ bis yₘ der Messwerte, die durch die Kamera 6 aufgenommen worden sind, für einen bestimmten Zielbereich bezeichnet. Werden diese mit den Beleuchtungsparametern verknüpft, so ergibt sich ein n-Tupel von Startwerten für Bestrahlungsparameter 36, die mit z₁ bis zₒ bezeichnet werden und die beispielsweise die Größe des Fokusflecks des Lasers auf der Netzhaut, die Strahlungsintensität des Lasers, die Pulsdauer und/oder die Zahl der abgegebenen Pulse für einen oder mehrere Zielbereiche bezeichnen.

Ein Ziel der Erfindung besteht darin, optimierte Startwerte für Bestrahlungsparameter für jeden Zielbereich zu ermitteln. Dieses Ergebnis ist zunächst mit der Ermittlung der Startwerte erreicht, was durch die gestrichelte Trennlinie 25 in Figur 5 dargestellt ist. Darauf kann ein tatsächlicher Laserbetrieb des Lasers 2 und damit eine Behandlung der Netzhaut erfolgen, und es kann danach der Einfluss der Laserbehandlung auf die Netzhaut durch Wiederholung des beschriebenen Vorgangs, das heißt Beleuchtung der Netzhaut mit definierten Parametern und Ermittlung eines Kamerabilds oder einer Aufnahme, durchgeführt werden. Das Ergebnis des erneut aufgenommenen Kamerabilds kann mit dem ursprünglich aufgenommenen Kamerabild abgeglichen und eine Differenz ermittelt werden, die die Wirkung der Behandlung durch den Laser 2 zeigt. Entspricht die Wirkung des Lasers bei der Behandlung nicht dem zu erreichenden Behandlungsziel, so kann einerseits der Algorithmus 35 korrigiert beziehungsweise trainiert werden, und andererseits kann aus den Beleuchtungsparametern bei der zweiten Beleuchtung und den mit dem zweiten Kamerabild aufgenommenen Messdaten ein neuer Satz von Bestrahlungsparametern ermittelt werden.

In Figur 6 ist zunächst der Ablauf der Verfahrensschritte 20 bis 24 entsprechend Figur 2 dargestellt, worauf auf den Schritt der Behandlung 24 ein weiterer Schritt 21' einer Beleuchtung der Netzhaut mit definierten Beleuchtungsparametern und gleichzeitig oder versetzt die Aufnahme eines Kamerabilds in einem Schritt 22' erfolgt, worauf die Beleuchtungsparameter und die mit dem Kamerabild aufgenommenen Messdaten in einem weiteren Schritt 23' zu neuen Bestrahlungsparametern miteinander verknüpft werden.

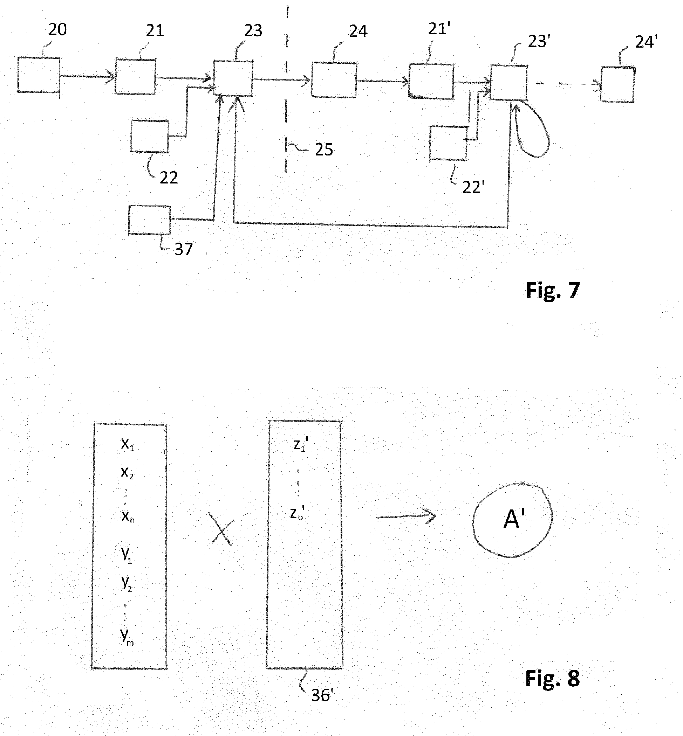

In Figur 7 ist ein Ablaufdiagramm dargestellt, in dem die Struktur des Ablaufdiagramms aus Figur 6 wiederholt ist, wobei sich an die erneute Verknüpfung 23' von den nach einer ersten Laserwirkung 24 berücksichtigten Beleuchtungsparametern und Messdaten der Kamera eine weitere Laserbehandlung 24' anschließt. Zudem werden die Informationen aus den im Korrekturverfahren ermittelten Bestrahlungsparametern aus dem Schritt 23' in einer Schleife zur Verbesserung des Verknüpfungsalgorithmus verwendet, der in den Verfahrensschritten 23 und 23' durchgeführt wird. Durch dieses Feedback von Korrekturgrößen kann der Verknüpfungsalgorithmus, der der Verknüpfung zwischen den Beleuchtungsparametern, den Messdaten der Kamera und den Startwerten für Bestrahlungsparameter zugrunde liegt, sukzessive verbessert werden. Zu diesem Zweck können Behandlungen ein und desselben Individuums oder von Gruppen von Individuen, beispielsweise mit ähnlichem Krankheitsbild, berücksichtigt werden, um den Algorithmus zu verbessern. Der Algorithmus kann zu diesem Zweck auch zentral gespeichert sein, beispielsweise auf einem Server oder in einer Cloud, und jeweils für individuelle Behandlungen abgerufen werden, wobei Korrekturgrößen dann an den Server beziehungsweise die Cloud zurückgespielt werden, um möglichst viele Trainingsdaten aus verschiedenen Behandlungsvorgängen an unterschiedlichen Orten zum Training des Algorithmus verwenden zu können.

In Figur 7 ist zudem angedeutet, dass in einem weiteren Verfahrensschritt 37 zusätzliche Parameter an dem individuellen Patienten beziehungsweise seinem Auge oder seiner Netzhaut gemessen werden, die zur Kalibrierung in den Verknüpfungsschritt 23 mit eingespeist werden. Hierzu können im Schritt 37 beispielsweise integrale Größen der Netzhaut, z. B. eine durchschnittliche Pigmentierung oder die Stärke der Färbung der Iris des Auges oder die Farbe der Iris oder die Stärke der Hautpigmentierung des Individuums oder die Farbe der Hautpigmentierung und/oder die Farbe und Stärke der Pigmentierung der Haare des Individuums mit einem Sensor, insbesondere einer Kamera, gemessen und in den Verknüpfungsschritt 23 eingespeist werden. Auch der Einfluss dieser Größen kann beim Training des Zuordnungsalgorithmus berücksichtigt oder bei einzelnen Behandlungen als Kalibrierparameter eingespeist werden.

Figur 8 zeigt schematisch die Entwicklung des Zuordnungsalgorithmus A, A' durch Verknüpfung von korrigierten Bestrahlungsparametern 36' mit den ursprünglichen Beleuchtungsparametern x₁ bis xₙ und den durch das Kamerabild ermittelten Messdaten y₁ bis yₘ. Vor dem einzelnen Trainingsschritt wurden den genannten Eingangsparametern die Startwerte 36 für Bestrahlungsparameter zugeordnet, und der Algorithmus erhält entsprechende Korrekturdaten 36' mit Bestrahlungsparametern z₁' bis zₒ', die als optimierte Ergebnisdaten interpretiert werden und den Algorithmus nachschärfen.

Durch dieses Verfahren wird nach der erforderlichen Anzahl von Trainingsdurchgängen eine Optimierung des Verknüpfungsalgorithmus und damit auch eine Optimierung von Startwerten für Bestrahlungsparameter bereits in einem ersten Schritt erreicht.

## Patentansprüche

1. Verfahren zur Ermittlung von Bestrahlungsparametern für den Betrieb eines Lasers (2) zur Bestrahlung von einem oder mehreren Zielbereichen der Netzhaut (16) eines Auges (4) mit einem Behandlungsstrahl (11), bei dem die Netzhaut ganz oder teilweise mit einer Beleuchtungseinrichtung (5, 5') unter definierten Beleuchtungsparametern beleuchtet wird und während und/oder kurz nach der Beleuchtung wenigstens eine Aufnahme, insbesondere wenigstens ein Kamerabild, zumindest eines Zielbereiches der Netzhaut erfasst wird und bei dem unter Verwendung des/der Aufnahmen oder Kamerabilder zumindest Startwerte der Bestrahlungsparameter für die Bestrahlung eines oder mehrerer Zielbereiche der Netzhaut ermittelt werden.

2. Verfahren nach Anspruch 1, **dadurch gekennzeichnet, dass** zur Ermittlung der Startwerte der Bestrahlungsparameter mittels der Aufnahme(n) oder des/der Kamerabilder die Intensität der Pigmentierung für einen oder mehrere Zielbereiche der Netzhaut und/oder die Intensitätsverteilung der Pigmentierung auf der Netzhaut ermittelt werden.

3. Verfahren nach Anspruch 1 oder 2, **dadurch gekennzeichnet, dass** zur Ermittlung der Startwerte der Bestrahlungsparameter für einen oder mehrere Zielbereiche der Netzhaut Korrekturwerte bezüglich der zu erwartenden Absorption des Behandlungsstrahls im Glaskörper des Auges auf dem Weg zu den zu bestrahlenden Zielbereichen ermittelt werden.

4. Verfahren nach Anspruch 1, 2 oder 3, **dadurch gekennzeichnet, dass** zur Erzeugung eines Kamerabildes die Netzhaut mit einer Strahlung mit bekannter Wellenlängenverteilung beleuchtet wird, die insbesondere eine Infrarotstrahlung und/oder Licht in einem durch Melanin absorbierten Wellenlängenbereich umfasst, wobei zusätzlich die Lichtstärke der Beleuchtungseinrichtung und/oder die auf eine Flächeneinheit der Netzhaut auftreffende gesamte Lichtleistung und/oder die auf eine Flächeneinheit der Netzhaut auftreffende Lichtleistung in einem definierten Wellenlängenbereich bestimmt wird und wobei die Helligkeitswerte und/oder Farben des Kamerabildes zur Ermittlung der Startwerte der Parameter für die Bestrahlung der Netzhaut verwendet werden.

5. Verfahren nach Anspruch 1, 2, 3 oder 4, **dadurch gekennzeichnet, dass** für einen oder mehrere Zielbereiche die Dicke der Netzhaut ermittelt und bei der Ermittlung der Startwerte der Bestrahlungsparameter berücksichtigt wird, wobei die Ermittlung der Dicke der Netzhaut insbesondere durch die Erstellung einer OCT-Dickenkarte der Netzhaut erfolgt, und/oder dass zur Ermittlung der Startwerte der Bestrahlungsparameter die Art und Intensität der Pigmentierung von von der Netzhaut verschiedenen Körperregionen der zu behandelnden Person, insbesondere die Art und Intensität der Pigmentierung der Iris des Auges oder der Haut oder der Haare, verwendet werden.

6. Verfahren nach einem der Ansprüche 1 bis 5, **dadurch gekennzeichnet, dass** zur Ermittlung der Startwerte der Bestrahlungsparameter außer der/den erfassten Aufnahmen oder Kamerabildern zusätzlich eine oder mehrere vorgegebene oder vorgebbare Referenzaufnahmen oder Referenzbilder verwendet werden.

7. Verfahren nach einem der Ansprüche 1 bis 6, **dadurch gekennzeichnet, dass** zur Ermittlung der Bestrahlungsparameter für einen oder mehrere zu bestrahlende Zielbereiche der Netzhaut unabhängig voneinander einerseits die Intensität der Pigmentierung des/der Zielbereich(e) und andererseits die zu erwartende Absorption des Behandlungsstrahls in der Augenlinse auf dem Weg zu den Zielbereichen ermittelt werden, indem wenigstens zwei Messungen unter verschiedenen Beleuchtungsbedingungen durchgeführt werden, wobei bei den zwei oder mehr Messungen insbesondere wenigstens einer der folgenden Parameter variiert wird: Größe des fokussierten Lichtflecks auf der Netzhaut, wobei jeweils die Abweichung von einer zu erwartenden Lichtintensitätsverteilung auf der Fläche des Fokusflecks gemessen wird, Einstrahlrichtung eines Beleuchtungsstrahls der Beleuchtungseinrichtung durch die Pupille und den Glaskörper zur Beleuchtung des jeweiligen Zielbereiches, Einstrahlrichtung zum Zielbereich und/oder Ausfallrichtung eines erfassten reflektierten Beleuchtungsstrahls der Beleuchtungseinrichtung, Wellenlängenbereich oder Wellenlängenverteilung eines Beleuchtungsstrahls der Beleuchtungseinrichtung.

8. Verfahren nach einem der Ansprüche 1 bis 7, **dadurch gekennzeichnet, dass** zur Ermittlung der Bestrahlungsparameter für einen oder mehrere zu bestrahlende Zielbereiche der Netzhaut die Intensität der Pigmentierung des/der Zielbereich(e) und/oder die zu erwartende Absorption des Behandlungsstrahls in der Augenlinse auf dem Weg zu den Zielbereichen ermittelt werden, indem wenigstens zwei Messungen durchgeführt werden, **dadurch gekennzeichnet, dass** eine der Messungen auf die Absorptions und/oder Reflektionscharakteristik im Bereich der Papille und/oder wenigstens eines Blutgefäßes oder eines Teils eines Blutgefäßes in der Netzhaut gerichtet ist, während eine zweite Messung auf die Absorptions und/oder Reflektionscharakteristik in einem der übrigen, zu bestrahlenden Bereiche der Netzhaut gerichtet ist.

9. Verfahren zum Training eines Algorithmus oder eines neuronalen Netzes zur Ermittlung der Startwerte von Bestrahlungsparametern gemäß einem der Ansprüche 1 bis 8, **dadurch gekennzeichnet, dass** für eine Mehrzahl von Einzelbehandlungen als Trainingsdaten einerseits aus Bildern ermittelte Datensätze von zu bestrahlenden Zielbereichen und die zugehörigen Beleuchtungsparameter sowie insbesondere jeweils zusätzlich erfasste Sekundärinformationen als Eingangsinformationen und andererseits die bei der jeweils nachfolgenden Laserbehandlung eingestellten Bestrahlungsparameter als Ergebnisgrößen miteinander verknüpft werden.

10. Verfahren nach einem der Ansprüche 1 bis 9, **dadurch gekennzeichnet, dass** bei der Ermittlung der Startparameter für alle Zielbereiche eine Kalibrierung mit einem oder mehreren der folgenden Kalibrierparameter der jeweils zu behandelnden Person durchgeführt wird: gemessene integrale Pigmentierungsstärke der Haut, gemessene integrale Pigmentierungsstärke der Iris des Auges, gemessene Pigmentierungsstärke der Haare, Farbe der Hautpigmentierung, Farbe der Iris, Farbe der Haare.

11. Verfahren zur Ermittlung von Bestrahlungsparametern für den Betrieb eines Lasers zur Bestrahlung von einem oder mehreren Zielbereichen der Netzhaut eines Auges mit einem Behandlungsstrahl, bei dem zunächst Startwerte der Bestrahlungsparameter nach einem Verfahren gemäß einem der Ansprüche 1 bis 10 ermittelt werden, bei dem darauf mindestens ein Zielbereich mit einem Titrationsstrahl oder einem Behandlungsstrahl mit den Startparametern bestrahlt wird, bei dem darauf wenigstens eine Fortsetzungsaufnahme oder ein Fortsetzungskamerabild des oder der mit dem Behandlungsstrahl bestrahlten Zielbereiche der Netzhaut erfasst wird und bei dem unter Verwendung des/der Fortsetzungsaufnahme(n) oder des/der Fortsetzungskamerabildes (-kamerabilder) und der Startwerte der Bestrahlungsparameter zumindest Fortsetzungswerte der Bestrahlungsparameter für die Bestrahlung eines oder mehrerer Zielbereiche der Netzhaut ermittelt werden.

12. Vorrichtung zur Bestrahlung von einem oder mehreren Zielbereichen der Netzhaut eines Auges mit einem Behandlungsstrahl, mit einem Laser zur Erzeugung des Behandlungsstrahls, mit einer Beleuchtungseinrichtung zur Beleuchtung von wenigstens einem oder mehreren Zielbereichen der Netzhaut mit definierten Beleuchtungsparametern sowie mit einer Aufnahmeeinrichtung, insbesondere einer Kamera, zur Erfassung wenigstens einer Aufnahme oder eines Bildes zumindest eines Zielbereiches der Netzhaut und mit einer Verarbeitungseinrichtung, die dazu eingerichtet ist, unter Verwendung der Beleuchtungsparameter und des/der Aufnahmen oder Kamerabilder zumindest Startwerte der Bestrahlungsparameter für die Bestrahlung eines oder mehrerer Zielbereiche der Netzhaut mit einem Behandlungsstrahl zu ermitteln.
